# EUROPEAN PATENT APPLICATION

(11) **EP 1 256 579 A1**
(43) Date of publication of application: **13.11.2002**
(21) Application number: 01904503.8
(22) Date of filing: 16.02.2001
(51) Int. Cl.: C07D 319/24, G01N 33/53

(54) **DIOXIN DERIVATIVES AND METHOD OF MEASUREMENT THEREWITH**

(30) Priority: 17.02.2000 JP 2000039914
(71) Applicant: OTSUKA PHARMACEUTICAL CO., LTD., Chiyoda-ku Tokyo 101-8535 (JP)
(72) Inventor: YANAIHARA, Noboru, SHIZUOKA-SHI, Shizuoka 420-0961 (JP); KODAIRA, Tsukasa, Itano-gun Tokushima 771-0220 (JP)
(74) Representative: Hartz, Nikolai F., Dr.
(86) International application number: JP0101118
(87) International publication number: WO01060812

(57) **Abstract**

The invention provides a highly sensitive measurement and detection technique for dioxins, which has advantages inherent to immunoassay; and markers to be used in the technique.

Disclosed are a biotinylated dioxin derivative of formula (1): 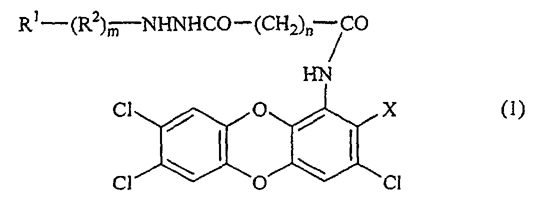 (wherein X represents a hydrogen atom or a chlorine atom; R¹ represents a biotin residue; R²'s, which may be identical to or different from one another, independently and individually represent an arginine residue or a lysine residue; n is an integer from 1 to 5 inclusive; and m is an integer from 1 to 3 inclusive), and an immunoassay method for dioxins characterized by using the derivatives as a marker.

## Description

### Technical Field

The present invention relates to a novel biotinylated dioxin derivative, and to an improved immunoassay method for dioxins, *inter alia*, 2,3,7,8-tetrachlorodibenzo-p-dioxin (hereinafter referred to as "2,3,7,8-TCDD"), making use of the derivative.

### Background Art

Dioxins collectively refer to polychlorinated dibenzo-p-dioxins (PCDDs) and their analogues, polychlorinated dibenzofurans (PCDFs). Dioxins include numerous isomers which differ from one another in terms of the positions and numbers of chlorine atoms. Dioxins are discharged from factories and incinerators when, for example, chlorine-containing organic compounds are incinerated, and spread into the air, rivers, and soil.

Dioxins have attracted attention because of their toxicity; in particular, 2,3,7,8-TCDD is highly toxic, adversely affecting organs and physiological functions of humans, livestock, and poultry, and raising serious social problems in both Japan and overseas countries.

Hitherto, dioxins have been measured and/or detected through methods employing physicochemical apparatus―such as high performance liquid chromatography, GC mass spectrometry, and LC mass spectrometry―which are routinely used for the measurement and detection of chlorine-containing organic compounds. However, such physicochemical methods involve drawbacks, in that the apparatus to be employed are expensive, sample volumes are limited, and sample throughput is limited. Therefore, demand remains for development of a method that replaces the conventional methods; i.e., a more convenient method that enables accurate, rapid measurement and/or detection of numerous samples.

Meanwhile, immunoassays generally have a variety of advantages, including reduced measurement time, simple and convenient procedure, low cost, and capability of assaying multiple specimens simultaneously. Therefore, attempts have been made to apply immunoassay techniques to measurement and/or detection of dioxins, and accordingly, production of so-called "specific antibodies" for use in such assay systems and development of assay systems making use of such specific antibodies have heretofore been studied (see, for example, Toxicology, 45, 229-243 (1987); Anal. Chem., 70, 1092-1099 (1988); U.S. Patent Nos. 4,238,472, 5,429,925, and 5,674,697; and Japanese Patent Application Laid-Open (*kokai*) Nos. 14691/1988 and 74494/1988.

However, these assay systems are not perfectly satisfactory for measurement and/or detection of dioxins, which are present at very low concentrations in specimens, and thus, in this technical field, establishment and provision of more sensitive immunoassays for dioxins are demanded (The Science of the Total Environment, 239, 1-18 (1999)).

The present invention contemplates solving the above-described problems and providing more sensitive immunoassays for dioxins. More particularly, an object of the present invention is to provide an improved method for measuring and/or detecting dioxins while maintaining the aforementioned advantages inherent to immunoassays. Another object of the invention is to provide a novel marker for use in the method.

### Disclosure of the Invention

The present inventors have conducted extensive studies, and have found that a novel biotinylated dioxin derivative of formula (1) below satisfies the above-mentioned objects and is useful as a marker for the immunoassay of dioxins. Moreover, through employment of the derivative, the present inventors have succeeded in establishing an assay system which realizes highly sensitive measurement and/or detection of dioxins as compared with that attained by conventional techniques. The present invention has been accomplished on the basis of these findings.

Accordingly, the present invention provides a biotinylated dioxin derivative of formula (1): 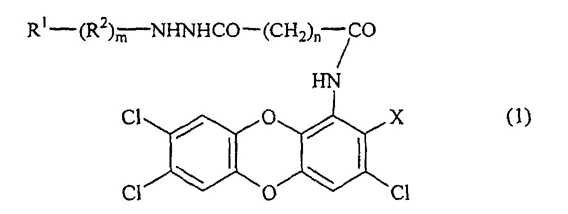 wherein X represents a hydrogen atom or a chlorine atom; R¹ represents a biotin residue; R²'s, which may be identical to or different from one another, independently and individually represent an arginine residue or a lysine residue; n is an integer from 1 to 5 inclusive; and m is an integer from 1 to 3 inclusive.

The present invention also provides an immunoassay method for dioxins characterized by comprising using the above-described biotinylated dioxin derivative as a marker.

The present invention also provides a reagent which contains the above-described biotinylated dioxin derivative and is useful for the immunoassay of dioxins.

### Brief Description of the Drawing

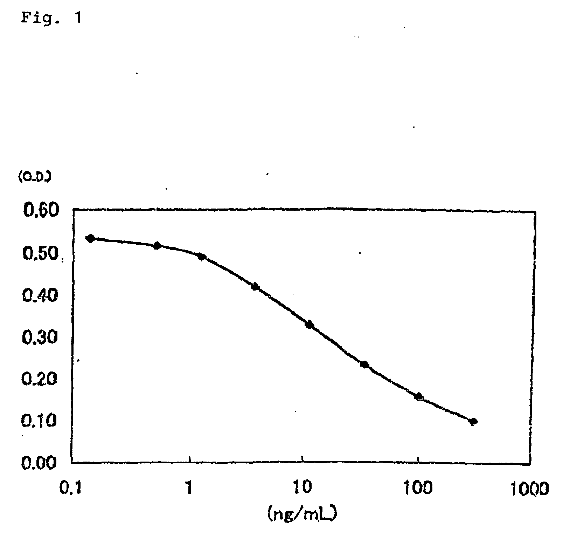

Fig. 1 shows a standard curve which was obtained in Example 2, step 5) and which is to be used in the immunoassay method of the present invention.

### Best Mode for Carrying Out the Invention

The biotinylated dioxin derivative of the present invention is represented by the above formula (1), wherein bonding between the biotin residue (R¹) and an amino acid residue (R²) which bonds directly to R¹ is an amide bonding between the carboxyl group of biotin and the amino group of amino acid. Moreover, unless otherwise specified, the amino acid residues represented by R² (i.e., Arg and Lys) encompass D-isomers and L-isomers thereof.

The biotinylated dioxin derivative of the present invention may be prepared through condensation reaction between a hydrazide of formula (2) and a compound of formula (3) :

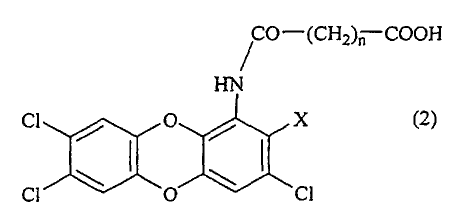

R¹―(R²)ₘ―OH (3)

wherein R¹, R², X, m, and n have the same meanings as described above.

The compound of formula (3) can be prepared through a generally employed peptide synthesis method, including routine liquid phase formats and solid phase formats. For example, when biotin and one or more desired species of amino acid are sequentially subjected to condensation reaction, a compound of formula (3) can be obtained. The condensation reaction performed in such a synthesis method may be any type of known condensation reaction method.

Examples of condensation reaction methods that can be employed in the above synthesis method include the azide method, the acid anhydride mixture method, the DCC (dicyclohexylcarbodiimide) method, the active ester method, the oxidation-reduction method, the DPAA (diphenylphosphoylazide) method, the DCC method in combination with an additive (1-hydroxybenzotriazole, N-hydroxysuccinamide, N-hydroxy-5-norbornene-2,3-dicarboxyimide, etc.), and the Woodward's method.

Solvents which are employed in the above methods may be appropriately selected from among a variety of solvents that are customarily used for peptide condensation reactions performed in this technical field. Examples of the solvents include, but are not limited to, N-methylpyrrolidone (NMP), dimethylformamide (DMF), dimethlsulfoxide (DMSO), and mixtures thereof.

When the condensation reaction is performed, functional groups that do not participate in reaction may be protected by ordinarily employed protecting groups through conventional methods, and deprotected after completion of reaction. Methods for protection and/or deprotection are known, and reagents used in such methods may be appropriately selected from among known ones.

Examples of protecting groups for amino include benzyloxycarbonyl, tert-butoxycarbonyl (Boc), isobornyloxycarbonyl, and p-methoxybenzyloxycarbonyl. Examples of protecting groups for carboxyl include lower alkyl esters such as methyl esters, ethyl esters, and tert-butyl esters; benzyl esters, and groups capable of forming p-methoxybenzyl esters.

Examples of protecting groups for the guanidino group of arginine include 2,2,5,7,8-pentamethylchroman-6-sulfonyl (Pmc), 2,2,4,6,7-pentamethyldihydrobenzofuran-5-sulfonyl (Pbf), nitro, benzyloxy carbonyl, Boc, and p-toluenesulfonyl.

These protecting groups can be removed through any conventional method; e.g., through catalytic reduction, or through a method employing liquid ammonia/sodium, hydrogen fluoride, hydrogen bromide, hydrogen chloride, trifluoroacetic acid (TFA), acetic acid, or methanesulfonic acid.

The hydrazide of formula (2) may be prepared by subjecting hydrazine protected by an arbitrary protecting group, such as Boc, benzyloxycarbonyl, 2,2,2-trichloroethoxycarbonyl (Troc), to condensation reaction with a compound of formula (2) in a manner similar to that described above, and subsequently, the protecting group of the resultant compound is removed.

The condensation reaction between the thus-prepared hydrazide of the compound of formula (2) and the compound of formula (3) may be carried out in a manner similar to that described above. Incorporation and removal of protecting groups are also carried out as described above.

Compounds of formula (2) are easily prepared in accordance with known methods (see, for example, Toxicology and Applied Pharmacology, 50, 137-146 (1979)).

The thus-prepared biotinylated dioxin derivatives of formula (1) of the present invention may be purified as desired, through any of a variety of routine purification procedures, such as high performance liquid chromatography.

The biotinylated dioxin derivatives of formula (1) of the present invention are characterized by their solubility to the liquid phase of an immunoassay system (i.e., water-solubility). Because of this feature, the formula (1) derivatives find remarkable utility as markers to be employed in immunoassay systems for dioxins.

Conventionally, biotin has been widely used as an indirect marker, on the basis of specific bonding between biotin and avidin. Having a relatively low molecular weight (MW: 244.31) as compared with general enzymes, biotin is preferred to be used as a marker in immunoassays. However, since biotin *per se* is insoluble to water, conjugates with dioxins which lack solubility in water are also insoluble to water, thus preventing use of biotin in the above-described immunoassays.

In contrast, the biotinylated dioxin derivatives of the present invention have water solubility, which enables them to be suitably applied to immunoassay of dioxins, and moreover, the derivatives exhibit water solubility without affecting their cross reactivity to anti-dioxin antibodies. From these points, the biotinylated dioxin derivatives of the present invention are suited for use in a long-hoped immunoassay system for dioxins.

A preferred embodiment of the assay method of the present invention will be described hereinbelow, and when the assay method is enzyme immunoassay, which is a preferred mode of the invention, dioxins on a level falling within a range of about 0.1-100 ng/mL can be assayed. This dioxin concentration range corresponds to that in, for example, human urine or plasma. Accordingly, the present invention provides a highly sensitive assay system which realizes measurement of dioxins contained in a sample such as a human urine sample.

The biotinylated dioxin derivatives of the present invention are useful as markers in various types of immunoassays performed for dioxins, including seven isomers of polychlorinated dibenzo-p-dioxins led by 2,3,7,8-TCDD and/or ten isomers of polychlorinated dibenzofurans led by 2,3,7,8-trichlorodibenzofuran. In particular, the derivatives are preferably employed for immunoassay of dioxins containing 2,3,7,8-TCDD as a target substance to be measured.

When X in formula (1) is hydrogen, the biotinylated dioxin derivatives of the present invention exhibit particularly low toxicity. Thus, the assay method of the present invention has an advantage of ensured safe operation during the assay, as it employs derivatives of such low toxicity. Biotinylated dioxin derivatives in which X is hydrogen, n is 4, and m is 2 are particularly preferred.

When biotinylated dioxin derivatives of the present invention are used in conventional immunoassays, such as enzyme immunoassays (EIAs) and enzyme immunometric assays (ELISAs), dioxins, *inter alia*, 2,3,7,8-TCDD, can be measured and/or detected specifically with high sensitivity.

The essential requirement in the immunoassay of the present invention that a biotinylated dioxin derivative of the present invention be used as a marker in the assay system. Excepting that, the immunoassay of the invention is performed in accordance with conventional immunoassay procedures.

The anti-dioxin antibody to be employed in the assay system may be immobilized onto a solid phase of an arbitrary type. The solid phase is any one that is conventionally employed in the art. No particular limitation is imposed on immobilization of the antibody onto the solid phase, and typical physical bonding or chemical bonding may be performed for immobilization.

No particular limitation is imposed on the immunoreaction, and the conditions therefor are conventional ones; generally, for example, the immunoreaction may be carried out at a temperature of 45°C or lower, typically at about 4°C to 40°C, for about 0.5 to several hours. No particular limitation is imposed on the solvent to be employed in the reaction and the pH of the solvent, so long as the reaction is not adversely affected thereby. Example solvents include citrate buffer, phosphate buffer, Tris buffer, and acetate buffer.

Preferably, the assay method of the present invention is carried out in a competitive format using the marker of the invention. For example, a sample which may contain dioxins to be measured is added to an assay system including an immobilized anti-dioxin antibody and a biotinylated dioxin derivative of the present invention, allowing antigen-antibody reaction (of competitive format) to proceed. Subsequently, the marker that has been bound to the immobilized antibody is measured by use of an ordinary detection reagent prepared from avidin.

No particular limitation is imposed on the detection reagent prepared from avidin, and there may be employed those generally employed in the art; e.g., streptoavidins and avidins which have been modified with a variety of markers. No particular limitation is imposed on the markers for avidin; any of conventionally known ones and those which may be used in future can be employed. Specifically, enzymes such as alkaline phosphatase (ALP) and peroxidase (HRP) are preferred. Avidin can be modified with any of these markers through a method known *per se*. The modified products may also be procured as commercial products.

As a marker antigen (standard), the compound of formula (2) may be used; in particular, compounds of formula (2) in which X is hydrogen and n is 4 are preferred.

A particularly preferred example assay method of the present invention that realizes specific measurement and/or detection of dioxins is ELISA of high sensitivity, which will be described in the Examples section hereinbelow.

In measurement and/or detection of dioxins performed according to the present invention, an assay kit comprising the biotinylated dioxin derivative of the present invention as an active component is conveniently employed. The kit may further comprise, in addition to the derivative, optional reagents which may be required for carrying out the measurement and/or detection, such as the aforementioned detection reagent prepared from avidin, anti-dioxin antibody, standard antigen, and assay buffer.

The assay method for dioxins according to the present invention provides effective means for measurement and detection of dioxins present as analytes in humans or animals; i.e., dioxins contained in samples from humans or animals (such as tissue, blood, urine, cerebrospinal fluid, and saliva samples), and tissue or blood of fish, and thus is useful for elucidation and research of effects exerted by exogenous substances on biostimulation - secretion mechanism or organs or tissues.

When the analytes are samples from rivers, the sea, waste water, air, or soil, the method of the present invention is useful for measuring or detecting dioxins in such corresponding environments.

No particular limitations are imposed on the anti-dioxin antibodies to be employed in the assay method of the present invention, so long as the anti-dioxin antibodies exhibit specific reactivity to the object to be measured; i.e., a dioxin, and therefore, they may be any of the aforementioned known antibodies or similarly produced antibodies. The antibodies encompass polyclonal antibodies, such as antisera from warm-blooded animals or hen's egg antibodies, and monoclonal antibodies.

A suitable antibody is selected in accordance with the dioxin to be measured. Preferably, the antibody has reactivity with 2,3,7,8-TCDD.

The present inventors have previously confirmed that polyclonal antibodies generally exhibit excellent titer against an antigen of a low-molecular organic compound, such as a dioxin, and therefore, use of polyclonal antibodies is preferred in the present invention.

The antibodies may be obtained through immunization of a warm-blooded animal (other than a human) with an immunogen. The method or procedure therefor is basically known *per se*.

The immunogen is preferably a conjugate product of compound of formula (2) and a carrier protein.

No particular limitation is imposed on the carrier protein, and there may be employed any of a variety of carrier proteins that are widely employed in the art by virtue of being acknowledged to enhance immunogenicity of an antigen or hapten, such as albumin, globulin, thioglobulin, and hemocyanin; and polylysin. Through a condensation reaction similar to that described above, these carrier proteins can be conjugated to a compound of formula (2) by use of a customary reagent.

The thus-obtained carrier protein conjugate may exhibit a desired immunogenicity by itself, and thus may be used as an immunogen for producing a specific antibody which may be suitably used in the assay method of the present invention.

If desired, the carrier protein conjugate may be adsorbed onto a polymer adsorbent, and the resultant adsorption product used as an immunogen.

The polymer adsorbent which may be employed as enhancing immunogenicity is any of a variety of polymer substances routinely employed, such as polyvinyl pyrrolidone, latex, serum protein such as butathioglobulin, and charcoal powder. The polymer adsorbent and the aforementioned carrier protein conjugate are mixed in accordance with a conventional method, to thereby yield an adsorbent of interest.

Immunization by use of the immunogen and preparation of antibodies of interest may be performed in accordance with conventional methods. For example, polyclonal antibodies may be prepared by injecting, generally a plurality of times, the above-mentioned immunogen in the form of an emulsion prepared by mixing the immunogen with a Freund's complete adjuvant to a warm-blooded animal such as a rabbit, sheep, guinea pig, or chicken, after which the resultant antiserum is collected through a conventional method. In the case of chicken, the immunogen is administered to a hen a plurality of times, so that an egg produced by the hen contains an immunoglobulin (IgY), and IgY is collected from the yolk of the egg through a routine method.

Alternatively, the antibodies may be obtained in the form of monoclonal antibodies. The monoclonal antibodies may be prepared, for example, as follows. The above-described immunogen is administered to a mouse together with Freund's complete adjuvant for immunizing the mouse and allowing the mouse to produce antibodies. Through, for example, a conventional method such as cell fusion, the antibody-producing cells are fused with bone marrow/spleen cells for cloning. A single clone cell that produces the antibody of interest is separated, and the cell is cultured.

The thus-prepared antibodies may be purified according to needs through any conventional method such as ammonium sulfate precipitation, ion chromatography, or affinity chromatography.

### Examples

The present invention will next be described in more detail by way of non-limiting examples, which should not be construed as limiting the scope of the present invention.

### Example 1

By use of the compound of formula (2) (X = hydrogen, n = 4) prepared in Referential Example 1 (described hereinafter) as a starting material, a biotinylated dioxin derivative of the present invention was prepared.

Briefly, water soluble carbodiimide (3.8 µL; Peptide Institute , Inc.) was added to a solution (1.0 mL) prepared by dissolving the above-described formula (2) compound (2.3 mg, 5.4 µmol) and Boc-N₂H₃ (1.4 mg, 10.7 µmol) in dimethylformamide at -10°C, and the resultant solution was stirred at room temperature for 18 hours. Distilled water was added to the solution under cooling with ice, and the solid matter was collected and dried under reduced pressure, to thereby yield 2.5 mg of 3,7,8-trichlorodibenzo-p-dioxin having Boc-NHNHCO(CH₂)₄CONH- at the 1-position.

This product was dissolved in trifluoroacetic acid (TFA, 0.5 mL), followed by stirring at room temperature for 40 minutes, to thereby remove the Boc group, and then subjected to condensation with biotinyl-L-Arg(pbf)-L-Arg(pbf)-OH (7.3 mg, 6.8 µmol) prepared in Referential Example 2 (described hereinafter) in DMF (1.0 mL) at room temperature for 2 days. This process is similar to the method described above; that is, the water soluble carbodiimide/1-hydroxybenzotriazole (WSCD/HoBt) method.

Distilled water was added to the resultant mixture under cooling with ice for solidification, and the resultant solid matter was collected and dried under reduced pressure. The dried product (5.0 mg) was dissolved in TFA (2.4 mL) containing distilled water (65 µL) and triisopropylsilane (65 µL), followed by stirring at room temperature for 2 hours. Diethyl ether was added thereto for solidification, and the solid matter was dried, to thereby yield 4.5 mg of a crude product of the target substance.

The crude product (4.5 mg) was diluted by 10% acetic acid (1 mL) and purified through fractional reverse HPLC by use of 0.1% TFA/acetonitrile solvent mixture having a concentration varying in accordance with the linear concentration gradient (85/15 → 20/80, 30 minutes), to thereby yield 0.2 mg of the target compound (the compound of formula (1), X = hydrogen, m = 2, n = 4, R² = L - Arg).

Mass spectrometry analysis revealed that a peak obtained coincided with a theoretical value (molecular weight: 983.378).

### Example 2

### 1) Preparation of antibody-immobilized plate

To each well of a 96-well microplate (Maxi Sorp, Nunk), an aliquot (100 µL) of goat anti-rabbit IgG serum (Jackson Immuno Reserch) which had been diluted 200-fold with 0.1M citrate buffer was dispensed. After the plate was allowed to stand at 25°C for 2 hours, the supernatant was discarded, and Block Ace (340 µL, Dainippon Pharmaceutical) was added to each well. The plate was allowed to stand at 25°C for 2 hours, and thereafter, washed 5 times with a washing solution (0.15 M NaCl containing 0.1% Tween 20). An anti-dioxin antibody solution (prepared in Referential Example 3, which will be described hereinafter) which had been diluted 1,000-fold with 0.01M PBS buffer (containing 0.05% BSA, 0.05% Tween 20, and 10mM EDTA) was added to each well in an amount of 100 µL. The plate was allowed to stand at 4°C for 24 hours, to thereby yield an antibody-immobilized plate to which the anti-dioxin antibody was immobilized.

### 2) Preparation of standard solutions

One mg of the compound of formula (2) (X = hydrogen, n = 4) prepared in Referential Example 1 was dissolved in hexamethylphospharamide or DMSO, and the resultant solution was diluted 1,000-fold with PBS buffer, to thereby obtain a standard solution having a concentration of 1 µg/mL. The thus-prepared solution was subjected to 5-fold serial dilution to thereby prepare diluted solutions having the following concentrations: 200, 40, 8, 1.6, 0.32, and 0.064 µg/mL.

### 3) Preparation of marker

A compound of the present invention (200 µg, compound of formula (1); X = hydrogen, m = 2, n = 4, R² = L - Arg) prepared in Example 1 was dissolved in 0.1N acetic acid. Upon use, the solution was diluted 1,000-fold with PBS buffer.

### 4) Preparation of color developing solution

Streptoavidin-HRP (200 µg, CALBIOCHEM) was diluted 2,000-fold with PBS buffer.

A color developing agent was prepared by dissolving an OPD tablet (10 mg; produced by Sigma) in 0.1M sodium sulfate - citrate buffer (pH 5.0; 10 mL) containing 0.015% hydrogen peroxide and adjusting the final concentration to 1 mg/mL.

### 5) Immunoassay

The wells of the anti-dioxin-immobilized plate were washed, and a standard sample (50 µL) prepared by the serial dilution or a sample to be assayed (50 µL) was added to each well, followed by addition of the above-described marker (50 µL). The microtiter plate was sealed for allowing reaction for 2 hours at room temperature. Thereafter, the contents of each well were discarded and washed 5 times.

The above-prepared color-developing solution (100 µL) was added to each well for allowing reaction for 15 minutes at room temperature. Subsequently, 2N sulfuric acid (100 µL) serving as enzyme reaction stopping solution was added to each well, and absorbance of the contents of each well was measured at 490 nm by use of an absorptiometer for microtiter plate.

The thus-obtained curve is shown in Fig. 1.

### Referential Example 1

1-Amino-3,7,8-trichlorodibenzo-p-dioxin (30.0 mg, 0.1 mmol) was added to an absolute pyridine solution (2.0 mL) containing 4-dimethylaminopyridine (6.1 mg, 0.05 mmol).

Monomethyl adipate (74.1 µL, 0.5 mmol) was dissolved to the absolute pyridine solution (2.0 mL), and thionyl chloride (360 µL, 5 mmol) was added dropwise thereto, followed by reflux under nitrogen for 5 hours. The solvent was evaporated, and the residue was dried.

The thus-obtained acid chloride was dissolved in absolute pyridine (1.0 mL), and the solution was added to the above-prepared pyridine solution containing 1-amino-3,7,8-trichlorodibenzo-p-dioxin, followed by stirring at room temperature for 3 days. The solvent was evaporated, and ether was added thereto for solidification. The resultant solid matter that precipitated was collected, whereby a target methyladipoyl derivative was obtained (14.0 mg/31.5%).

The methyladipoyl derivative (14.0 mg, 31.5 µmol) was suspended in 95% ethanol (5.0 mL), and 0.1N NaOH (378 µL, 378 µmol) was added thereto, followed by stirring at 70°C for 4 hours. After the solution was cooled to 0 to 4°C, 1N HCl was added thereto, and the resultant solution was subjected to extraction twice with ethyl acetate. The ethyl acetate layer was washed with 1N HCl, and then with saturated saline three times. The washed layer was dehydrated over sodium sulfate, and filtered, after which the solvent was evaporated. The residue was freeze-dried in the presence of dioxane, to thereby yield 10.0 mg (73.5%) of the target product (compound of formula (2); X = hydrogen, n = 4). Through HPLC, the purity of the target product was found to be 98% or more.

### Referential Example 2

Biotinyl-Arg(Pbf)-Arg(Pbf)-OH was synthesized in accordance with a routinely performed solid phase synthesis procedure.

Briefly, H-Arg(Pbf)-Trt(2-CL)-resin (1.0 g, 0.49 mmol) was washed with DMF, and condensed with Fmoc-Arg(Pbf)-OH (1.27 g, 1.96 mmol) by use of an HATU reagent (0.75 g, 1.96 mmol). The peptide resin was treated with a 50% solution (10 mL) of piperidine in DMF at room temperature for 20 minutes, to thereby remove Fmoc. In a similar manner, biotin (0.48 g, 1.96 mmol) was subjected to condensation treatment. The peptide resin was treated with a mixture (25 mL) of acetic acid, trifluoroethanol, and dichloromethane (1 : 2 : 7) at room temperature for 1 hour, to thereby liberate peptide from the resin. The resin was filtered off, and the filtrate was subjected to evaporation. Ether was added to the residue for solidification, whereby 540 mg ofthe target compound was obtained as a sediment.

HATU: 0-(7-azabenzotriazol-1-yl)-1,1,3,3-tetramethylunonium hexafluorophosphate

### Referential Example 3

The compound prepared in Reference Example 1 was employed as a hapten and caused to be condensed with Keyhole limpet hemocyanin (KLH, Pierce), whereby a conjugate to be used for immunization was prepared.

Briefly, an aliquot of the compound prepared in Reference Example 1 (5.2 mg) was dissolved in 20% isopropanol-containing phosphate buffer (1 mL), and mixed with KLH (20 mg) which had been dissolved in 2 mL of 0.1M PBS (pH 6.0). Water soluble carbodiimide (100 mg, Peptide Institute, Inc.) was added thereto, and the resultant solution was allowed to react at room temperature for 30 minutes.

The resultant reaction mixture (3 mL) was mixed with 50% polyvinylpyrrolidone (3 mL, Merck), and the mixture was divided into 3 aliquots. To each aliquot, an equiamount of Freund's complete adjuvant (1 mL, Calbiochem) was added for emulsification. The emulsion was subcutaneously injected to 3 rabbits (Japanese white, male; body weight 2.0-2.5 kg) so that the amount of the immunogen administered was 0.3 mg/rabbit. The animals were boosted by being given half the amount of the first injection, and boosting was repeated 6 times at 2-week intervals. One week after the final immunization, whole blood of rabbit was collected, and allowed to stand at 37°C for 1 hour, then at 4°C overnight, followed by centrifugal separation at 3,000 rpm, whereby antisera having a sufficient titer were obtained. The antisera were freeze-dried for storage.

The thus-produced antisera were studied regarding their titer and cross-reactivity with dioxins, revealing that all antiserum samples obtained from any of the 3 rabbits exhibit dioxin-specific reactivity.

1-amino-3,7,8-trichlorodibenzo-p-dioxin or 1-amino-2,3,7,8-tetrachlorodibenzo-p-dioxin was immobilized onto a column (FMP-activated Cellulofine; Seikagaku Corporation), and the above-obtained sera were applied to a column for affinity chromatography.

Through affinity chromatography (eluant: 1M acetic acid), there was obtained an antibody preparation characterized by having specific reactivity with 3,7,8-trichlorodibenzo-p-dioxin and/or 2,3,7,8-tetrachlorodibenzo-p-dioxin, and substantial absence of cross-reactivity with dibenzo-p-dioxin and chlorophenol-containing substances.

## Claims

1. A biotinylated dioxin derivative of formula (1): 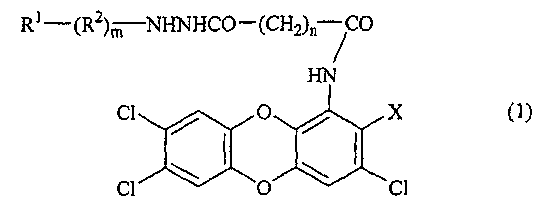 wherein X represents a hydrogen atom or a chlorine atom; R¹ represents a biotin residue; R²'s, which may be identical to or different from one another, independently and individually represent an arginine residue or a lysine residue; n is an integer from 1 to 5 inclusive; and m is an integer from 1 to 3 inclusive.

2. The biotinylated dioxin derivative according to claim 1, wherein X represents a hydrogen atom, n is 4, and M is 2.

3. An immunoassay method for dioxins **characterized by** comprising using as a marker a biotinylated dioxin derivative of formula (1): 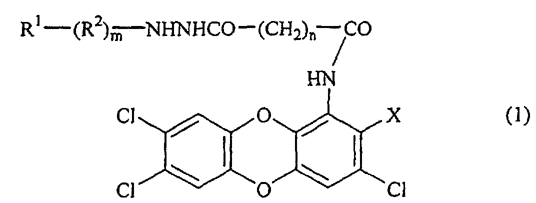 wherein X represents a hydrogen atom or a chlorine atom; R¹ represents a biotin residue; R²'s, which may be identical to or different from one another, independently and individually represent an arginine residue or a lysine residue; n is an integer from 1 to 5 inclusive; and m is an integer from 1 to 3 inclusive.

4. A reagent for immunoassay of dioxins, containing a biotinylated dioxin derivative of formula (1): 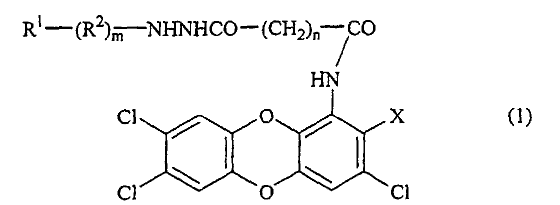 wherein X represents a hydrogen atom or a chlorine atom; R¹ represents a biotin residue; R²'s, which may be identical to or different from one another, independently and individually represent an arginine residue or a lysine residue; n is an integer from 1 to 5 inclusive; and m is an integer from 1 to 3 inclusive.
